# EUROPÄISCHE PATENTANMELDUNG

(11) **EP 0 595 788 A2**
(43) Veröffentlichungstag der Anmeldung: **04.05.1994**
(21) Anmeldenummer: 94100613.2
(22) Anmeldetag: 18.01.1994
(51) Int. Cl.: G02B 21/08, A61B 19/00

(54) **Operationsmikroskop mit einer Beleuchtungseinrichtung**

(30) Priorität: 28.04.1993 DE 9306412 U; 29.09.1993 DE 9314578 U
(71) Anmelder: J.D. Möller Optische Werke GmbH, D-22880 Wedel (DE)
(72) Erfinder: Twisselmann, Lorenz, Dr.-Ing., D-25497 Prisdorf (DE)
(74) Vertreter: Richter, Werdermann & Gerbaulet

(57) **Zusammenfassung**

Ein Operationsmikroskop für ophthalmologische Anwendungen umfaßt eine Beleuchtungseinrichtung, die über ein außerhalb der optischen Achse des Hauptobjektives (19) angeordnetes Umlenkelement (16) Licht einstrahlt, das in das Zentrum des Sehstrahlenganges Licht einstrahlt, so daß die Koaxialbeleuchtung des Operationsfeldes verbessert wird.

## Beschreibung

Die Erfindung betrifft ein Operationsmikroskop mit einer Beleuchtungseinrichtung, die über ein Umlenkelement Licht einstrahlt, die über ein außerhalb der optischen Achse des Hauptobjektivs angeordnetes Umlenkelement Licht einstrahlt.

Eine wesentliche Bedeutung hat hierbei die Beleuchtung des Operationsfeldes, die in nichtophtalmologischen Anwendungen, beispielsweise in der Hals-Nasen-Ohren- und Neurochirurgie, in Form einer achsnahen Schrägbeleuchtung gefordert wird.

Bei mikrochirurgischen Eingriffen am Auge wird hingegen angestrebt, das Beleuchtungslicht senkrecht, d.h. in der optischen Achse des Mikroskopobjektivs auf das Operationsfeld zu richten. Diese Beleuchtungsart hat den Vorteil, daß die senkrecht einfallenden Lichtstrahlen von der Netzhaut diffus reflektiert werden und die Linsenkapsel, das ist die Umhüllung der Augenlinse, durch das regrediente Licht in einem rötlichen Durchlicht erscheinen lassen. Dadurch werden Gewebereste, die nach Entfernen der Augenlinse abgesaugt werden müssen, kontrastreich sichtbar gemacht.

In der Praxis sind jedoch bisher nur solche Beleuchtungseinrichtungen bekannt geworden, bei denen nur ein kleiner Bruchteil des Beleuchtungsstrahlenbündels senkrecht einfällt, d.h. in Richtung des Sehstrahlenganges, während der größere Teil achsnah als Schräglicht einfällt.

Eine Beleuchtungseinrichtung wird beispielsbweise in der DE 40 28 605 beschrieben. Während bei Operationen in nichtophthalmologischen Fachrichtungen, insbesondere in der Hals-Nasen-Ohren- und Neurochirurgie das Operationsfeld mit achsnaher Schrägbeleuchtung ausgeleuchtet wird, wird bei mikrochirurgischen Eingriffen am Auge, insbesondere bei Katarakt-Operationen angestrebt, das Beleuchtungslicht senkrecht, d.h. in der optischen Achse des Mikroskopobjektes auf das Operationsfeld zu richten. Diese Beleuchtungsart hat den Vorteil, daß die senkrecht einfallenden Lichtstrahlen von der Netzhaut diffus reflektiert werden und die Linsenkapsel, das ist die Umhüllung der Augenlinse, durch das regrediente Licht in einem rötlichen Durchlicht erscheinen lassen. Dadurch werden Gewebereste, die nach Entfernen der Augenlinse abgesaugt werden müssen, kontrastreich sichtbar gemacht. Die DE 40 28 605 legt eine Aufgabenstellung dar, bei ophthalmologischen Operationen das Patientenauge nur zu einem Bruchteil mit senkrecht einfallendem Licht, zum anderen Teil mit achsnaher einfallendem Schräglicht zu beleuchten. Hierzu wird ein erstes, vor der optischen Achse des Mikroskopobjektivs angeordnetes Umlenkelement so gestaltet, daß es nur einen Teil des Beleuchtungslichtes in achsnaher Schrägbeleuchtung zum Objektpunkt hinlenkt und daß ein zweites Umlenkelement in oder hinter der optischen Achse des Mikroskopobjektivs angeordnet ist, welches einen anderen Teil des Beleuchtungslichtes senkrecht oder achsnäher als das erste Umlenkelement zum Objektpunkt lenkt. Als Neigungswinkel des Lichtes zur optischen Achse werden beim ersten Umlenkelement und beim zweiten Umlenkelement bis zu 4^{o} angestrebt. Das zweite Umlenkelement kann senkrecht zur optischen Achse verschiebbar sein. Zur Variation der durchgelassenen Lichtmengen können verstellbare Blenden vorgesehen werden. In einer speziellen Ausführungsform sind die Umlenkelemente der Gestalt, daß das erste als Spiegel ausgebildete Umlenkelement im unteren Bereich einen mittigen Ausschnitt in Form einer Aussparung aufweist, durch den das durchgelassene Licht auf einen zweiten Spiegel fallen kann, der zwischen den beiden durch die Zoom-Objektive definierten Beobachtungsstrahlengängen liegt.

In der DE-U1 93 06 412.8 wird zur Verbesserung der Koaxialbeleuchtung des Operationsfeldes vorgeschlagen, durch das Umlenkelement in das Zentrum des Sehstrahlenganges Licht einzustrahlen.

In der Praxis werden mit den vorstehend beschriebenen Anordnungen jeweils teilweise Überdeckungen von Beleuchtung und Betrachtung auf der Netzhaut erreicht, so daß auch die Pupille des beobachteten Auges nur teilweise leuchtend (Rotreflex) erscheint.

Es ist daher Aufgabe der vorliegenden Erfindung, das eingangs genannte Operationsmikroskop, insbesondere für ophthalmologische Anwendungen, derart weiterzubilden, daß die Koaxialbeleuchtung des Operationsfeldes verbessert wird, wobei die Überdeckung verbessert und eine Gleichmäßigkeit des Rotreflexes erzielt wird.

Diese Aufgabe wird durch das im Anspruch 1 beschriebene Operationsmikroskop gelöst, das erfindungsgemäß dadurch gekennzeichnet ist, daß das Umlenkelement in das Zentrum des Sehstrahlenganges Licht einstrahlt. Bei dieser Ausbildung einer gleichen Beleuchtungs- und Betrachtungsachse oberhalb des Objektes (Auges) wird das von der Retina zurückgestrahlte Licht als sogenannter Rotreflex, das ist die deutlich rötlich aufleuchtende Augenpupille, gut sichtbar.

Eine weitere Lösung der Aufgabe besteht nach Anspruch 8 darin, daß die Zahl und Anordnung der Umlenkelemente eine Gesamtreflektionsfläche bildet, die die Zoom-Eingänge im wesentlichen vollständig umschließt. Durch die Vielzahl der Umlenkelemente oder eine entsprechend ausgebildete Spiegelfläche wird die Patientenpupille gleichmäßig ausgeleuchtet.

Weiterbildungen der Erfindung mit den Merkmalen des Anspruches 1 bestehen darin, daß zwischen dem Zoom-Objektiv, vorzugsweise jedes der beiden Zoom-Objektive, eines binokularen Mikroskopes, und dem Hauptobjektiv ein Umlenkelement für einen von außerhalb der optischen Achse des Hauptobjektives eingestrahlten Beleuchtungsstrahlenbündels vorgesehen. Durch diese Maßnahme wird zum einen ein relativ kurzer Weg des Lichtes zum Objekt geschaffen, zum anderen sind keine besonderen Vorkehrungen in dem Mikroskoptubus oder den Mikroskoptuben erforderlich.

Nach einer weiteren Ausbildung der Erfindung ist das Umlenkelement ein unter 45^{o} zur optischen Achse geneigter Spiegel oder ein Prisma. Vorzugsweise wird folgende Anordnung gewählt: Durch eine Bohrung eines Beleuchtungsspiegels wird ein Lichtstrahlenbündel durchgelassen, das auf zwei unter 45^{o} zur Lichtstrahlenbündelachse geneigte Spiegelflächen eines Prismas oder eines Spiegelpaares fällt, wo das Lichtstrahlenbündel in zwei in entgegengesetzte Richtung laufende Strahlenbündel geteilt wird, von denen jedes auf eines der Umlenkelemente fällt, von wo es in die Richtung der optischen Achse oder parallel, aber achsennah zur optischen Achse gelenkt wird. Kleinere Abweichungen von der 45^{o} Stellung sind zulässig, sofern die Lichtstrahlen im wesentlichen noch im Zentrum des Sehstrahlenganges senkrecht auftreffen. Bei der genannten Ausführungsform bildes das Mikroskopobjektiv die Blende der Mikroskopbeleuchtung über dem Beleuchtungsspiegel außeraxial und über die beiden geneigten Spiegelflächen koaxial zu den Sehstrahlengängen oder dem Sehstrahlengang. Wird vor der geneigten Spiegelfläche eine Sammellinse angebracht, kann eine im Brennpunkt dieser Linse angebrachte Blende mit dem Objektiv im Sehfeld abgebildet werden. Dies ist insbesondere dann von Vorteil, wenn zur Minimierung von Reflexen auf der Hornhaut in der Blende des Hauptbeleuchtungsstrahlenganges ein Bereich mit zerstreuender Wirkung gegeben ist, der nur einen extrem kleinen Reflex verursacht. Dieser Bereich wird dann von der genannten Sammellinse nicht abgebildet.

Zum Ein- und Ausschalten des Rotreflexes kann eine einstellbare Blende vorgesehen sein, die in einer Achse drehbar gelagert oder auch längsverschieblich ist und die vorzugsweise von einem Exzenter oder einem Hebel bewegt wird.

In einer ersten Ausführungsform der Erfindung mit den Merkmalen des Anspruches 8 sind in Richtung des Beleuchtungsstrahlenganges mehrere Umlenkelemente hinter- und/oder nebeneinander gestaffelt angeordnet, was den Vorteil hat, daß entsprechend viele Neigungswinkel der Umlenkelemente getrennt einstellbar sind. Um eine achsnahe Ausleuchtung zu bekommen, werden das erste Umlenkelement vor, das zweite in oder kurz hinter und alle weiteren Umlenkelemente hinter der optischen Achse angeordnet.

Zweckmäßigerweise sind alle Umlenkelemente in Richtung des Beleuchtungsstrahlenganges betrachtet vor dem Hauptobjetiv, vorzugsweise alle der optischen Achse nahen Umlenkelemente zwischen den Zoom-Objektiven und dem Hauptobjektiv angeordnet
Wie grundsätzlich nach dem Stand der Technik bekannt, weist ein Teil der Umlenkelemente Aussparungen oder Bohrungen zum Durchlaß des Beleuchtungslichtes auf die dahinter angeordneten Umlenkelemente auf.

In einer weiteren Ausführungsform besitzt das erste Umlenkelement eine in der Breite kleinere Verlängerung, die in den Zwischenraum zwischen den Beobachtungsstrahlengängen hineinragt. Die von dieser Verlängerung reflektierten Lichtstrahlen stellen die der optischen Achseo nächstliegenden Strahlen dar. Ebenso, wie das erste (oder die ersten Umlenkelemente) den vorderen Bereich vor der optischen Achse und vor den Beobachtungsstrahlengängen liegenden Bereich abdecken, sind in entsprechender Weise außerhalb der Beobachtungsstrahlengänge hintere Umlenkelemente vorgesehen, welche Licht in Richtung der Pupille reflektieren. Hierdurch ergibt sich eine nahezu vollständige Umschließung der Zoom-Eingänge.

Nach einer weiteren Ausgestaltung lenkt das erste Umlenkelement das Beleuchtungslicht unter einem kleineren Neigungswinkel zur optischen Achse als die weiteren Umlenkelemente. Damit wird durch das erste Umlenkelement ein Beleuchtungsstrahlenbündel geschaffen, das achsparalleler als die Strahlenbündel der übrigen Umlenkelemente verläuft. Der betreffende Neigungswinkel des vom ersten Umlenkelementes reflektierten Strahlenbündels zur optischen Achse beträgt 2^{o} oder weniger, der Neigungswinkel der übrigen Lichtstrahlenbündel zur optischen Achse liegt unter 3^{o}.

Als Umlenkelement wird ein unter 45^{o} zur optischen Achse geneigter Spiegel oder ein Prisma gewählt, wobei entsprechend dem einzustellenden Neigungswinkel Abweichungen zulässig sind. Zur Abdeckung des Lichtstrahlenbündels, d.h. zum Ein- und Ausschalten des Rotreflexes kann eine einstellbare Blende vorgesehen sein, die in einer Achse drehbar gelagert oder auch längsverschieblich ist und die vorzugsweise von einem Exzenter oder einem Hebel bewegt wird. Nach einer weiteren Ausführungsform der Erfindung sind die Umlenkelemente quer zur optischen Achse verschiebbar.

Ausführungsbeispiele der Erfindung sind in den Zeichnungen dargestellt. Es zeigen
Fig. 1a, 1b, 1c jeweils ein Operationsmikroskop einer ersten Ausführungsform in verschiedenen Ansichten,
Fig. 2a, 2b jeweils unterschiedliche Ansichten des Beleuchtungsspiegels mit Prisma und dazwischen liegender Sammellinse bei einem Operationsmikroskop gemäß Fig. 1 a,
Fig. 3a, b und Fig. 4a, b jeweils verschiedene Ansichten unterschiedlicher Operationsmikroskope weiterer Ausführungsformen.

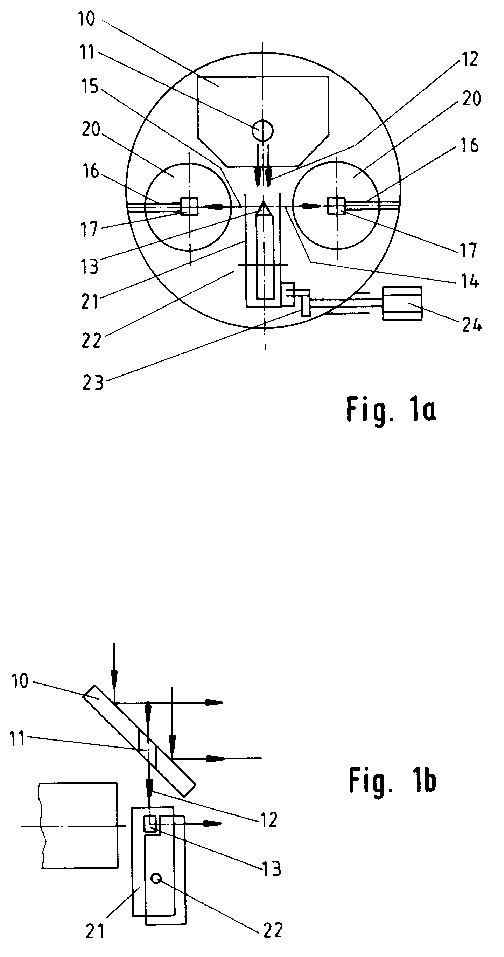

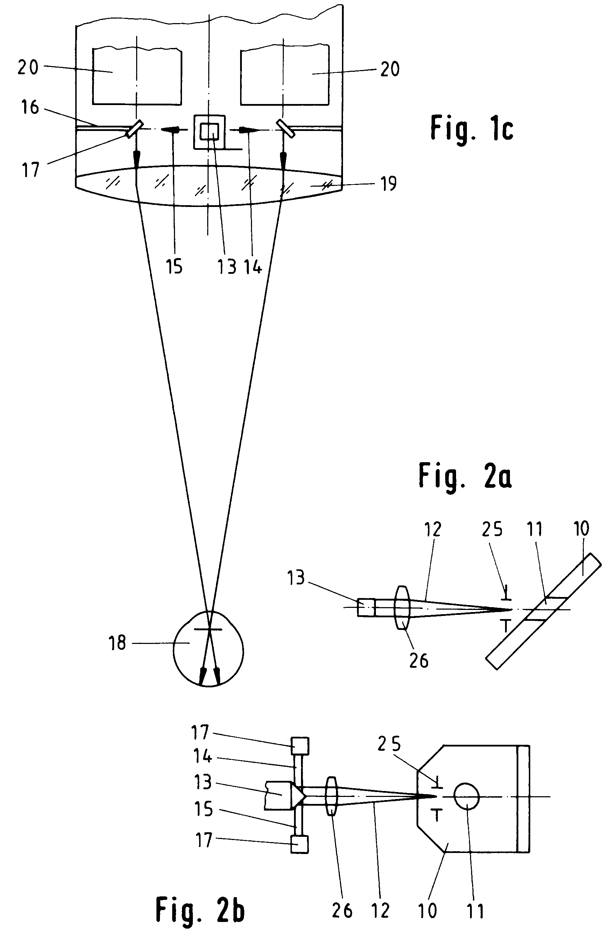

Das in den Fig. 1a bis 1c dargestellte Operationsmikroskop besitzt einen Beleuchtungsspiegel 10, der eine Bohrung 11 aufweist, durch welche auftreffendes Licht in Form eines Strahlenbündels 12 durchgelassen wird. Die Bohrung 11 kann auch als nicht reflektierende Teilfläche des Beleuchtungsspiegels 10 ausgebildet sein. Die den Beleuchtungsspiegel 10 bestrahlende Beleuchtungsquelle ist der Übersicht halber fortgelassen. Das Lichtstrahlenbündel 12 fällt auf die reflektierenden Flächen 13 eines Prismas, die in einem Winkel von 90^{o} zueinander und bezogen auf die Richtung des Lichtstrahlenbündels 12 unter 45^{o} geneigt sind. Von den Spiegelflächen 13, die das Lichtstrahlenbündel 12 teilen, gelangen Teilstrahlenbündel 14 und 15, die in entgegengesetzten Richtungen laufen, auf Umlenkelemente 16 mit reflektierenden Flächen 17, von wo die Teillichtstrahlenbündel senkrecht zur Zeichenebene in Richtung des zu betrachtenden Objektes (s. Fig. 1) - Auge 18 abgelenkt werden. Die Umlenkelemente 16 können jeweils ein Prisma oder ein Spiegel sein. Diese Umlenkelemente 16 befinden sich zwischen dem Hauptobjektiv 19 und der Zoom-Einrichtung 20 eines jeden Strahlenganges des binokularen Mikroskopes. Zum Ein- und Ausschalten des Rotreflexes ist im vorliegenden Fall das Prisma mit den Spiegelflächen 13 mit einer Blende 21 abdeckbar, die in einer Achse 22 drehbar gelagert oder auch längsverschieblich ist. Hierzu dient ein Exzenter 23, der über eine Verstelleinrichtung 24 betätigbar ist. Die entsprechenden Grenzstellungen sind Fig. 1b zu entnehmen.

Eine weitere Variante zeigen Fig. 2a und 2b. Hier sind zwischen dem Beleuchtungsspiegel 10 und dem 90^{o} Prisma mit den Spiegelflächen 13 eine Blende 25 und eine Sammellinse 26 zur Aperturbildung für das lichtstrahlenbündel 12 bzw. eine Parallelausrichtung vorgesehen.

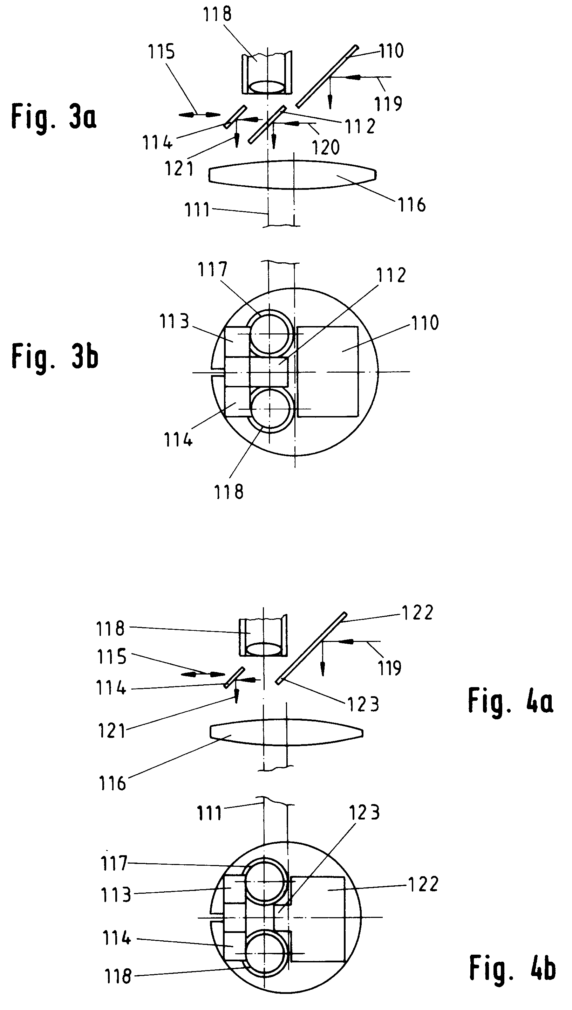

Das in Fig. 3a und b dargestellte Operationsmikroskop besitzt einen Spiegel 110 als erstes Umlenkelement, der vor der optischen Achse 111 angeordnet ist. In Richtung des einfallenden Beleuchtungslichtes gesehen sind unterhalb der Unterkante des Spiegels 110 weitere Umlenkelemente angeordnet, nämlich ein im wesentlichen in der optischen Achse liegender weiterer Umlenkspiegel 112 sowie weitere Umlenkspiegel 113 und 114 angeordnet, die seitlich versetzt hinter der optischen Achse und neben dem vorgenannten Spiegel 112 liegen. Zumindest die hinteren Spiegel 113 und 114 sind quer zur optischen Achse (siehe Doppelpfeil 115) verschiebbar. Die Umlenkspiegel 112, 113, 114 liegen zwischen dem Hauptobjektiv 116 und den Zoom-Objektiven 117 und 118.

Wie aus Fig. 3a und b erkennbar, werden eingestrahlte Lichtbündel 119, 120 und 121 in Richtung des (nicht dargestellten) Objektes gelenkt, wobei die Lichtbündel vor, zwischen und hinter den Zoom-Objektstrahlengängen liegen.

Bei der Ausführungsvariante nach Fig. 4a und b sind gleiche Teile mit gleichen Bezugszeichen wie in Fig. 2 versehen. Im Unterschied zu dieser Anordnung besitzt jedoch der erste Spiegel 122 eine in der Breite kleinere Verlängerung 123, die bis in den Bereich zwischen den durch die Zoom-Objektive 117 und 118 definierten Strahlengangbereich ragt. Anders ausgedrückt, die in Fig. 3a und b dargestellten Flächen 110 und 112 sind zu einer einzigen Fläche 122, 123 zusammengefaßt.

Der Winkel zwischen den Lichtstrahlen von der ersten reflektierenden Fläche 110 oder 122 und der Mittelachse 111 beträgt in dem geschilderten Ausführungsbeispiel 2^{o}. Der entsprechende Winkel der Lichtstrahlen, die von den weiteren reflektierenden Flächen 113 und 114 kommen, relativ zur optischen Achse 111 ist gegrenzt durch die Vignettierung der Beobachtungsöffnungen der entsprechenden reflektierenden Flächen und beträgt etwa 2,6^{o}. Der erste Winkel ist somit kleiner als der zweite, d.h. die Hauptmenge des in Richtung der Pupille gelenkten Lichtes verläuft unter geringerer Schrägstellung zur optischen Achse.

## Patentansprüche

1. Operationsmikroskop mit einer Beleuchtungseinrichtung, die über ein Umlenkelement Licht einstrahlt, über ein außerhalb der optischen Achse des Hauptobjektives (19) angeordnetes Umlenkelement (16) Licht einstrahlt, dadurch gekennzeichnet, daß die Beleuchtungseinrichtung über ein außerhalb der optischen Achse des Hauptobjektives (19) angeordnetes Umlenkelement (16) Licht einstrahlt, wobei das Umlenkelement (16) in das Zentrum des Sehstrahlenganges Licht einstrahlt.

2. Operationsmikroskop nach Anspruch 1, dadurch gekennzeichnet, daß zwischen dem Zoom-Objektiv (20), vorzugsweise jedes der beiden Zoom-Objektive eines binokularen Mikroskopes, und dem Hauptobjektiv (19) ein Umlenkelement (16) für ein von außerhalb der optischen Achse des Hauptobjektives (19) eingestrahltes Beleuchtungsstrahlenbündel (14, 15) vorgesehen ist.

3. Operationsmikroskop nach Anspruch 2, dadurch gekennzeichnet, daß das Umlenkelement (16) ein unter 45^{o} zur optischen Achse geneigter Spiegel (17) oder ein Prisma ist.

4. Operationsmikroskop nach Anspruch 2 oder 3, dadurch gekennzeichnet, daß ein Beleuchtungsspiegel (10), eine Bohrung (11) zum Durchlaß eines Lichtstrahlenbündels (12) aufweist, das auf zwei unter 45^{o} zur Lichtstrahlenbündelachse geneigte Spiegelflächen (13) eines Prismas oder eines Spiegelpaares fällt, wo das Lichtstrahlenbündel (12) in zwei in entgegengesetzte Richtung laufende Strahlenbündel (14, 15) geteilt wird, von denen jedes auf eines der Umlenkelemente (16) fällt, von wo es in die Richtung der oder parallel, aber achsnah zur optischen Achse gelenkt wird.

5. Operationsmikroskop nach Anspruch 4, dadurch gekennzeichnet, daß vor der geneigten Spiegelfläche (13) eine Sammellinse (26) angeordnet ist, deren Brennpunkt in der Ebene der Bohrung (11) des Beleuchtungsspiegels (10) liegt.

6. Operationsmikroskop nach einem der Ansprüche 1 bis 5, dadurch gekennzeichnet, daß vor den geneigten Spiegelflächen (13) eine einstellbare Blende (25) zur Abdeckung des Lichtstrahlenbündels vorgesehen ist.

7. Operationsmikroskop nach einem der Ansprüche 1 bis 6, dadurch gekennzeichnet, daß eine Blende (21) zur Abdeckung der Strahlenbündel (14, 15) drehbar und/oder längsverschlieblich gelagert ist, vorzugsweise in einem Exzenter (23) oder Hebel.

8. Operationsmikroskop mit einer Beleuchtungseinrichtung, die über ein Umlenkelement Licht einstrahlt, dadurch gekennzeichnet, daß die Beleuchtungseinrichtung über ein erstes vor der optischen Achse des Mikroskopobjektives angeordnetes Umlenkelement (110, 122) einen Teil (119) des Beleuchtungslichtes zum Objektpunkt hinlenkt und den anderen Teil (120, 121) des Beleuchtungslichtes über ein zweites in oder hinter der optischen Achse des Mikroskopobjektives (116) angeordnetes verschiebbares Umlenkelement zum Objektpunkt lenkt, wobei die Zahl und Anordnung der Umlenkelemente (110, 112, 113, 114, 122) eine Gesamtreflektionsfläche bildet, die die Zoom-Eingänge (117,118) im wesentlichen vollständig umschließt.

9. Operationsmikroskop nach Anspruch 8, dadurch gekennzeichnet, daß in Ricbntung des Beleuchtungsstrahlenganges betrachtet mehrere Umlenkelemente (110, 122, 112, 113, 114) hinter- und/oder nebeneinander gestaffelt angeordnet sind.

10. Operationsmikroskop nach einem der Ansprüche 8 und 9, dadurch gekennzeichnet, daß das erste Umlenkelement (110) vor, das zweite (112) in oder hinter und alle weiteren Umlenkelemente (113, 114) hinter der optischen Achse (111) angeordnet sind.

11. Operationsmikroskop nach einem der Ansprüche 8 bis 10, dadurch gekennzeichnet, daß alle Umlenkelemente (110, 122, 112, 113, 144) in Richtung des Beleuchtungsstrahlenganges betrachtet vor dem Hauptobjektiv (116) vorzugsweise alle der optischen Achse (111) nahen Umlenkelemente (112, 123) zwischen den Zoom-Objektiven (117, 118) und dem Hauptobjektiv (116) angeordnet sind.

12. Operationsmikroskop nach einem der Ansprüche 8 bis 11, dadurch gekennzeichnet, daß ein Teil der Umlenkelemente (110, 122) Aussparungen oder Bohrungen zum Durchlaß des Beleuchtungslichtes (120, 121) auf die dahinter angeordneten Umlenkelemente (112, 113, 114) aufweist.

13. Operationsmikroskop nach einem der Ansprüche 8 bis 12, dadurch gekennzeichnet, daß das erste Umlenkelement (122) eine in der Breite kleinere Verlängerung (123) aufweist, die in den Zwischenräumen zwischen den Beobachtungsstrahlengängen hineinragt.

14. Operationsmikroskop nach einem der Ansprüche 8 bis 13, dadurch gekennzeichnet, daß die in Richtung des Beleuchtungsstrahlenganges betrachtet hinteren Umlenkelemente (113, 114) außerhalb der Beleuchtungsstrahlengänge liegen.

15. Operationsmikroskop nach einem der Ansprüche 8 bis 14, dadurch gekennzeichnet, daß das erste Umlenkelement (110, 122) das Beleuchtungslicht unter einem kleineren Neigungswinkel zur optischen Achse (11) lenkt als die weiteren Umlenkelemente (113, 114).

16. Operationsmikroskop nach Anspruch 15, dadurch gekennzeichnet, daß das erste Umlenkelement das Beleuchtungslicht unter einem Neigungswinkel von 2^{o} oder weniger ablenkt und der Neigungswinkel im übrigen unter 3^{o} liegt.

17. Operationsmikroskop nach einem der Ansprüche 8 bis 16, dadurch gekennzeichnet, daß das Umlenkelement (110, 122, 123, 112, 113, 114) ein unter 45^{o} zur optischen Achse geneigter Spiegel oder ein Prisma ist.

18. Operationsmikroskop nach einem der Ansprüche 8 bis 17, dadurch gekennzeichnet, daß vor oder unter den geneigten Reflektionsflächen der Umlenkelemente (110, 122, 123, 112, 113, 144) einstellbare Blenden zur Abdeckung des Lichtstrahlenbündels vorgesehen sind.

19. Operationsmikroskop nach einem der Ansprüche 8 bis 18, dadurch gekennzeichnet, daß die Umlenkelemente (mit Ausnahme des ersten Umlenkelementes) und/oder die Blende zur Abdeckung der Strahlenbündel (119, 120, 121) drehbar und/oder längsverschieblich gelagert sind, vorzugsweise in einem Exzenter oder Hebel.
